# EUROPEAN PATENT APPLICATION

(11) **EP 3 925 949 A1**
(43) Date of publication of application: **22.12.2021**
(21) Application number: 20382533.6
(22) Date of filing: 19.06.2020
(51) Int. Cl.: C07C 247/04, C07C 247/08, C12Q 1/34, C12Q 1/6886, G01N 33/574, G01N 33/68, G01N 33/92

(54) **AZIDO COMPOUNDS AND METHOD FOR MONITORING ACID CERAMIDASE ACTIVITY IN INTACT CELLS USING THEM**

(71) Applicant: Consejo Superior de Investigaciones Científicas (CSIC), 28006 Madrid (ES); Universitat de Barcelona, 08028 Barcelona (ES)
(72) Inventor: CASASAMPERE FERRER, Mireia, 08034 Barcelona (ES); IZQUIERDO GARCIA, Eduardo, 08034 Barcelona (ES); CASAS BRUGULAT, Josefina, 08034 Barcelona (ES); ABAD SAIZ, José Luis, 08034 Barcelona (ES); FABRIAS DOMINGO, Gemma, 08034 Barcelona (ES); DELGADO CIRILO, Antonio, 08028 Barcelona (ES)
(74) Representative: Pons

(57) **Abstract**

The application relates to azido compounds of formula (I) and a method of using them together with a fluorogenic reporter as a click agent for the specific determination of acid ceramidase activity in intact cells. The application also relates to a kit to carry out said method and the use thereof for the diagnosis of a disease caused by the deregulation of ceramidase activity selected from Farber disease and cancer.

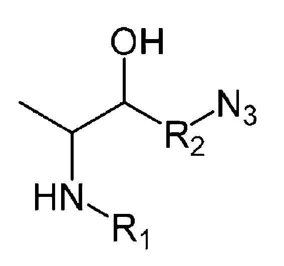

## Description

The invention relates to azido compounds, and more particularly, to ω-azido derivatives of deoxysphinganine and a method (*in* vitro) using thereof for the specific determination of acid ceramidase (hereiafter, AC) activity in intact cells. Also, the invention relates to a kit to carry out said method and the use thereof.

### STATE OF THE ART

Sphingolipids are a family of serine-derived lipids that exhibit not only important structural roles in membrane organization and dynamics, but also crucial cell signalling functions. *Ceramides* are the *hub* of sphingolipid metabolism. They can be produced by three different routes, the *de novo* pathway, hydrolysis of complex sphingolipids, and the salvage pathway. Ceramide biosynthesis *de novo* begins with the serine palmitoyltransferase (SPT) catalysed conjugation of L-serine with palmitoyl-CoA to produce 3-ketosphinganine, which is subsequently reduced to sphinganine and *N*-acylated to dihydroceramides by ceramide synthases (CerS). Besides producing sphingolipids with the canonical 2-amino-1,3-diol structure, several mutated forms of SPT show an alternative activity towards other amino acids, such as L-alanine, resulting in the formation of 1-deoxysphinganine or spisulosine (DOXSa). These mutations and the consequent accumulation of DOXSa and its downstream metabolites are the underlying cause of the rare Hereditary Sensory and Autonomic Neuropathy type 1 (HSAN1). By lacking the C1-hydroxyl group typical of sphingolipids, the *N*-acylated forms of DOXSa, namely 1-deoxydihydroceramides (DOXDHC), cannot be desaturated by the canonical dihydroceramide desaturase 1. Although unsaturated forms of DOXSa and DOXDHC are found endogenously, the double bond is located at Z-14. Furthermore, the absence of the C1-hydroxyl group precludes the formation of complex sphingolipids and of sphingosine-1-phosphate, the essential catabolic intermediate in the canonical degradation of sphingolipids. Instead, catabolism of DOXSa and DOXdhCer has been reported to occur by enzymes of the P450 family to produce mostly polyunsaturated and polyhydroxylated forms of DOXSa.

In addition to synthesis, ceramides are degraded to ethanolamine phosphate and a fatty aldehyde. This degradation takes place by sequential hydrolysis of the amide function, phosphorylation of the resulting free base and final irreversible retroaldol cleavage of the free base phosphate. Hydrolysis of the amide function is catalyzed by ceramidases, which are encoded by five different genes and are differentiated by the pH required for optimal activity (acid, neutral and alkaline). Whether the amide hydrolysis by ceramidases occurs on DOXdhCer is so far unknown.

Several reports sustain the involvement of ceramidases in the development of malignancies and in the failure to their treatment. Some examples include the role of acid ceramidase (AC) in melanoma (J. Leclerc, D. et al., Oncogene 2018, 38, 1282-1295), prostate cancer (J. C. Cheng, A. et al., J Clin Invest. 2013, 123, 4344-4358) and acute myeloid leukemia (S.-F. Tan, etal., Oncotarget 2016, 7, 83208-83222), the implication of the neutral ceramidase (NC) in colorectal cancer (M. Garcia-Barros, et al., FASEB J. 2016, 30, 4159-4171) and the contribution of alkaline ceramidase 3 (ACER3) to hepatocellular carcinoma and acute myeloid leukemia.[C. Chen, Biochem. Biophys. Res. Commun. 2016, 478, 33-8). On the other hand, mutations in the genes encoding for AC and ACER3 have been discovered to cause rare inherited diseases. One such condition is Farber disease, linked to mutations in the AC encoding gene (F. P. S. Yu, Orphanet J. Rare Dis. 2018, 13,121). The clinical interest of these amidases highlights the importance of developing specific probes of application in diagnostic and library screening for drug discovery.

Although several fluorescence-based methods for monitoring AC activity have been published (E. M. Saied, Chem. Phys. Lipids 2016, 197, 60-68) their use in flow cytometry analysis of labelled cells has not been disclosed.

The present invention proposes a simple, fast, robust and sensitive novel assay for the specific determination of AC activity in intact cells, preferably by flow-cytometry, based in the use of azido compounds and fluorogenic reporters.

The main advantage of this method over the known fluorogenic methods (Bedia C. et al. J Lipid Res 2010; 51: 3542-3547; Bhabak K.P. Bioorg Med Chem 2012; 20: 6154-61;Bhabak K.P. et al. Chembiochem 2013; 14: 1049-52 is the specificity for AC. In contrast, the previously reported fluorogenic methods are not specific because the substrates are also used by other ceramidases, such as the neutral ceramidase and the alkaline ceramidase ACER3. Consequently, although specificity can be achieved in vitro by selecting the appropriate pH (4,5 for AC, 7,0 for NC and 9,0 for ACER3), the three enzymes are measured when using intact cells. Conversely, since RBM5-177 (a compound within the scope of the present invention) is only recognized as a substrate by AC, the method described here allows the reliable determination of AC activity in intact cells. Furthermore, the method of the present invention can be optimized to be faster than the above fluorogenic method reported (30 min vs ≥3 h) and the readout is very persistent. Furthermore, using cells with a high degree of AC expression, the method of the present invention may be used to follow up the traffic of deoxysphingolipids, which can be of relevance in the study of conditions linked to altered deoxysphingolipid metabolism. Finally, the method of the invention allows the robust and long lasting AC activity independent lysosome staining in live cells.

### SUMMARY OF THE INVENTION

The inventors of the present invention have found that deoxydihydroceramides are exclusively hydrolysed by acid ceramidase. Using N-octanoyl-18-azidodeoxysphinganine as probe and a bodipy substituted bicyclononyne as fluorogenic reporter (both compounds form a labelling system), they show that this click strategy stains lysosomes predominantly, although extra lysosomal labelling (including mitochondria) is also observed in conditions of acid ceramidase overexpression. Using pharmacological and genetic tools, and high resolution mass spectrometry, the inventors have demonstrated that both lysosomal and extra lysosomal staining are acid ceramidase dependent. The application of the labelling system described here to the monitoring of acid ceramidase activity in intact cells is reported.

A first aspect of the present invention relates to compounds of formula (I) 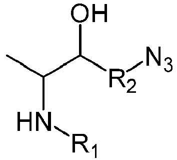 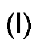 characterized in that:
R₁ is -CO-(C3-C20) alkyl, -CO-(C3-C20) alkenyl, -CO-(C3-C20) alkynyl, or CO-R₃-Y-
   R₄, where Y is selected from S, O, NH, SO, SO₂, CO, NHCO, CONH, COO and OCO,
   R₃ is (C1-Cₐ) alkylene, (C2-Cₐ) alkenylene or (C2-Cₐ) alkynylene,
   R₄ is (C1-C_{b}) alkyl, (C2-C_{b}) alkenyl or (C2-C_{b}) alkynyl, being a+b≤20, and a
   and b are both different to zero,
R₂ is a C4-C18 linear alkylene, C4-C18 linear alkenylene or a C4-C18 linear alkynylene.

In a preferred embodiment, the compound of formula (I) is the stereoisomer: 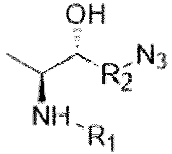 R1 and R2 are as defined above.

In a preferred embodiment, R₁ is -CO-(C3-C20) alkyl, more preferably R₁ is -COlinear (C3-C20) alkyl, even more preferably -CO-C7 alkyl, and even more preferably, R₁ is -CO(CH₂)₆CH₃.

In a preferred embodiment, R₂ is a C4-C18 linear alkylene, more preferably, is a C12 linear alkylene.

In another preferred embodiment, the compound of formula (I) is:

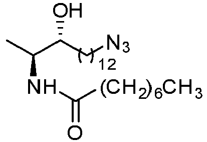

The term "alkyl" refers, in the present invention, to linear or branched saturated hydrocarbon radical, having preferably 3 to 20 carbon atoms, and which bind to the rest of the molecule by a single bond, for example, n-propyl, i-propyl, n-butyl, terc-butyl, sec-butyl, n-pentyl, etc. The alkyl groups may be optionally substituted by one or more substituents such as halogen, hydroxyl, alkoxyl, carboxyl, carbonyl, cyano, acyl, alkoxycarbonyl, amino, nitro, mercapto and alkylthio.,but preferably, the alkyl groups according to the present invention are unsubstituted.

The term "alkenyl" as used herein refers to a linear or branched, unsubstituted or substituted hydrocarbon radical containing at least one C-C double bond and having preferably 3 to 20 carbon atoms. The alkenyl groups may be optionally substituted by one or more substituents such as halogen, hydroxyl, alkoxyl, carboxyl, carbonyl, cyano, acyl, alkoxycarbonyl, amino, nitro, mercapto and alkylthio.,but preferably, the alkenyl groups according to the present invention are unsubstituted.

The term "alkynyl" as used herein refers to a linear or branched, unsubstituted or substituted hydrocarbon radical containing at least one C-C triple bond and having preferably 3 to 20 carbon atoms. The alkynyl groups may be optionally substituted by one or more substituents such as halogen, hydroxyl, alkoxyl, carboxyl, carbonyl, cyano, acyl, alkoxycarbonyl, amino, nitro, mercapto and alkylthio.,but preferably, the alkynyl groups according to the present invention are unsubstituted.

The term "alkylene" refers to a bivalent saturated aliphatic acyclic hydrocarbon group which may be linear or branched, such as methylene, ethylene, n-propylene, n-butylene, n-hexylene and the like. In the particular case of linear alkylene, it refers to a straight-chain without any carbon-atom branching. The alkylene groups may be optionally substituted by one or more substituents such as halogen, hydroxyl, alkoxyl, carboxyl, carbonyl, cyano, acyl, alkoxycarbonyl, amino, nitro, mercapto and alkylthio.,but preferably, the alkylene groups according to the present invention are unsubstituted.

The term "alkenylene" refers to a bivalent unsaturated aliphatic acyclic hydrocarbon group which may be linear or branched and comprises at least one carbon-to-carbon double bond. In the particular case of linear alkenylene, it refers to a straight-chain without any carbon-atom branching. The alkenylene groups may be optionally substituted by one or more substituents such as halogen, hydroxyl, alkoxyl, carboxyl, carbonyl, cyano, acyl, alkoxycarbonyl, amino, nitro, mercapto and alkylthio.,but preferably, the alkenylene groups according to the present invention are unsubstituted.

The term "alkynylene" refers to a bivalent unsaturated aliphatic acyclic hydrocarbon group which may be linear or branched and comprises at least one carbon-to-carbon triple bond. In the particular case of linear alkynylene, it refers to a straight-chain without any carbon-atom branching. The alkynylene groups may be optionally substituted by one or more substituents such as halogen, hydroxyl, alkoxyl, carboxyl, carbonyl, cyano, acyl, alkoxycarbonyl, amino, nitro, mercapto and alkylthio.,but preferably, the alkynylene groups according to the present invention are unsubstituted.

The compounds of the present invention represented by the formula (I) may include isomers, depending on the presence of multiple bonds (for example Z, E), including optic isomers or enantiomers, depending on the presence of chiral centers. The individual isomers, enantiomers or diastereoisomers and the mixtures of the same fall within the scope of the present invention. The individual enantiomers or diastereoisomers as well as their mixtures may be separated by means of conventional techniques.

The compounds of the invention may be in crystalline form like free compounds or like solvates and it is intended for both forms to be within the scope of the present invention.

Unless otherwise indicated, the compounds of the invention also include compounds which differ only in the presence of one or more isotopically enriched atoms. For example, compounds which have said structure, with the exception of the substitution of a hydrogen for a deuterium or for tritium, or the substitution of a carbon for a ^{13C} or ¹⁴C enriched carbon or a ¹⁵N enriched nitrogen, they are within the scope of this invention.

A second aspect of the invention refers to an *in vitro* method for the monitoring of acid ceramidase activity in intact cells, characterized in that it comprises:
a) contacting the intact cells with a compound of formula (I) defined in the first aspect of the invention,
b) washing the cells and adding fresh medium comprising a fluorogenic reporter to obtain stained cells, and
c) washing the cells and measuring the fluorescence emission of the stained cells obtained in step b),
wherein the fluorescence measured is indicative of the acid ceramidase activity.

Generally, the measurement of fluorescence is compared with a standard to detect a significant deviation from said standard, thus determining acid ceramidase activity. The standard is preferably a cell line with high expression of the enzyme or a line that lacks the acid ceramidase enzyme.

In step a) the compound of formula (I) is hydrolysed by the acid ceramidase contained in the intact cells, giving rise to the corresponding amine (I') resulting from the rupture of the NH-R₁ bond in the compound of formula (I). 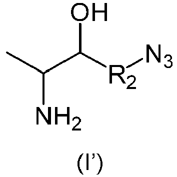 R₂ is as defined above.

In step b) the compound of formula (I') obtained in step a) reacts with the fluorogenic reporter via a copper (I) catalysed azide-alkyne cycloaddition click reaction. The click reaction covalently attaches an alkyne fluorogenic reporter to the azide group of the compound of formula I to develop a triazole ring, thereby resulting in detectable fluorescence. This way, stained cells are obtained.

In step c), the compound obtained by the reaction of compound of formula (I') and the fluorogenic reporter, that is present in the stained cells, are detected by fluorescence. The fluorescence can be measured by Thin Layer Chromatography Fluorescence (Fluorescence TLC), HPLC-fluorescence, fluorescence spectroscopy, confocal fluorescence microscopy and flow cytometry, among others.

In case of using Fluorescence-TLC or HPLC-fluorescence or in any procedure requiring a chromatographic separation, a cell lysis step is carried out before measuring fluorescence. The lysis step can be carried out, for example, suspending the cells in the appropriate volume of a 0.25 M saccharose solution with the proteases inhibitors aprotinin (preferably 1 mg/ml), leupeptin (preferably 1 mg/mml) and PMSF (Phenylmethylsulfonyl fluoride) (preferably 100 mM). The suspension was submitted to sonication (preferably three cycles of a 5 s at 10 watts/5s resting on ice). The cell lysate is centrifuged, preferably at 600 g for 5 min.

In a preferred embodiment, the step c) is carried out by flow cytometry. The use of flow cytometry avoids lysis stage since, as it is a specific substrate, it allows measuring the activity directly in cells. In addition, it can be adapted to high throughput flow cytometry.

The term "fluorogenic reporter" refers to a small molecule capable of supporting a chemical reaction that produces a fluorescence reporting molecule. This molecule absorbs energy of a specific wavelength and emits it in a given one of greater wavelength (ie, with less energy), and in which the amount of energy emitted (quantum yield) depends on both the small molecule itself and its chemical environment. As the skilled person in the art knows, a small molecule is a low molecular weight organic compound, typically involved in a biological process as a substrate or product. molecules within a mass range of 50 - 1000 daltons (Da). For the purpose of this invention the fluorogenic reporter contains a triple bond moiety capable to react via a copper (I) catalyzed azide-alkyne cycloaddition click reaction. The fluorogenic reporter can be, without limitation, a derivative of BODIPY, coumarin, acridine, anthracene, aloficocianine, cyanine, fluorescamine, fluorescein, xanthone and rhodamine. Preferably, the fluorogenic reporter is a derivative of BODIPY (boron dipyrromethene difluoride) group. Preferably, the fluorogenic reporter comprises a bicyclononyne moiety.

In a more preferred embodiment, the fluorogenic reporter is a BODIPY-allynyl derivative, more preferably the BODIPY-labelled bicyclononyne CO-1 (herein after, referred as CO-1):

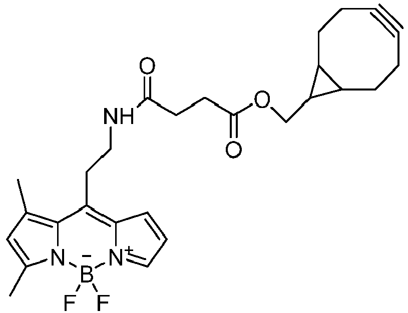

The term "intact" cells means a cell which maintains cell membrane integrity.

In a preferred embodiment, the cells are contacted with the compound of formula (I) in step a) between 10 and 240 min.

In another preferred embodiment, the concentration of compound of formula (I) in the medium in step a) is between 1 and 50 µM for a number of cells between 20,000 and 1,000,000.

In another preferred embodiment, the cells are contacted between 1 and 90 min and more preferably between 10 and 60 min with the fluorogenic reporter.

In another preferred embodiment, the concentration of the fluorogenic reporter is between 1 and 50 µM for a number of cells between 20,000 and 1,000,000.

In a preferred embodiment of the method, R₁ is -CO-C7 alkyl, more preferably, R₁ is -CO(CH₂)₆CH₃.

In a preferred embodiment of the method, R₂ is a C4-C18 linear alkylene, more preferably, is a C12 linear alkylene.

Another aspect of the invention refers to a kit for monitoring acid ceramidase activity in intact cells characterized in that it comprises a compound of formula (I) and a fluorogenic reporter. The compound of formula (I) and a fluorogenic reporter are as defined above. Also, the kit may include other components and reagents necessary to carry out sample monitoring.

Another aspect of the invention refers to the use of the kit for *in vitro* monitoring the acid ceramidase activity in intact cells.

Another aspect of the invention refers to the use of the kit for the diagnosis of a disease caused by the deregulation of ceramidase activity in an isolated sample of a subject, said disease selected from Farber disease and cancer. The cancer is preferably selected from the list comprising melanoma, prostate cancer, glioblastoma and acute myeloid leukemia.

In Farber disease, the probability an individual suffers from Farber disease, increases when acid ceramidase activity in the matter sample is approximately 25% lower than the ceramidase activity of a control sample of a healthy individual.

The isolated sample of the subject is preferably selected from blood, serum and tissue. The cells can be isolated from these samples according to any procedure known by the skilled person in the art.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings are provided by way of illustration and are not intended to be limiting of the present invention.

### DESCRIPTION OF THE DRAWINGS

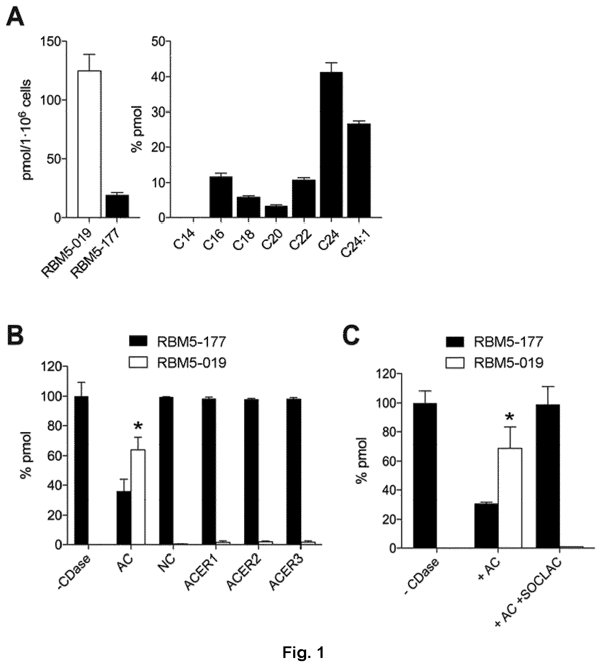

**Fig. 1****.** Metabolization of RBM5-177. A) A549 cells were incubated with RBM5-177 (5 µM, 30 min) and the lipid extracts were analyzed by UPLC-HRMS. Data (mean ± SD) were obtained from two experiments with triplicates. B) Lysates from A375/+Dox cells, recombinant NC or microsomes from ACER1, ACER2 or ACER3 overexpressing cells were incubated with RBM5-177 (5 µM, 30 min) in the appropriate buffer, and the lipid extracts were analyzed by UPLC-HRMS. Data (mean ± SD) were obtained from two different experiments with duplicates. Data corresponding to RBM5-019 were analysed by one way ANOVA followed by Dunnett's multiple comparison post-test if ANOVA < 0.05 (*, P < 0.001 from control without enzyme, - CDase). C) Lysates from A375/+Dox cells were incubated with RBM5-177 (5 µM, 30 min) prior treatment with SOCLAC (5 µM, 1 h) and the lipid extracts were analyzed by UPLC-HRMS. Data (mean ± SD) were obtained from two experiments with duplicates. Data corresponding to RBM5-019 were analysed by one way ANOVA followed by Dunnett's multiple comparison post-test if ANOVA P < 0.05 (*, P < 0.001 from - CDase). The right panel in A corresponds to the acyl chain of reacylated RBM5-019 (C14, tetradecanoyl, etc.).

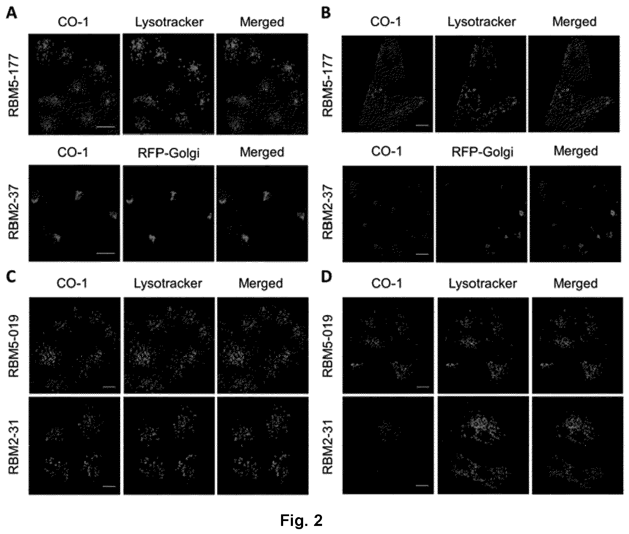

**Fig. 2****.** Co-localization studies of the indicated probes in A549 cells. **A, C**) A549 cells (incubated or not with RFP-Golgi were treated with the indicated probe (5 µM, 20 min), washed and incubated with CO-1 (1 µM, 10 min). **B, D**) After the probe treatment, cells were washed and incubated with medium for 5 h, washed and incubated with CO-1 (1 µM, 10 min). Lysotracker (75 nM) was added 30 minutes before fluorescence confocal microscopy (scale bar: 10 µm). Pearson's (P) and Manders' (M1 and M2) coefficients: **A**) RBM5-177-Lysotracker (P=0.802; M1=0.866; M2=0.744) and RBM2-37-RFP-Golgi (P=0.615; M1=0.588; M2=0.804). **B**) RBM5-177-Lysotracker (P=0.746; M1=0.822; M2=0.711) and RBM2-37-RFP-Golgi (P=0.346; M1=0.210; M2=0.206). **C**) RBM5-019-Lysotracker (P=0.713; M1=0.670; M2=0.774) and RBM2-31-Lysotracker (P=0.731; M1=0.768; M2=0.570). **D**) RBM5-019-Lysotracker (P=0.762; M1=0.735; M2=0.688).

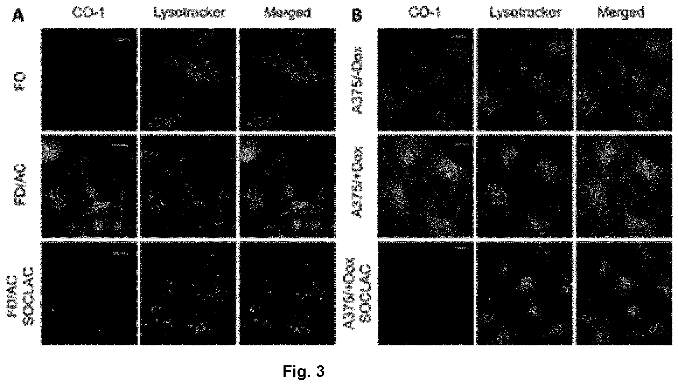

**Fig. 3****.** AC activity-dependence of labeling with RBM5-177. **A**) FD and FD/AC cells were incubated with RBM5-177 (5 µM, 4 h) prior treatment with SOCLAC 5 µM or vehicle for 1 h in FD/AC. **B**) A375 cells stimulated (+Dox) or not (-Dox) for AC overexpression were incubated for 20 min with RBM5-177 (5 µM, 10 min) prior treatment with SOCLAC 5 µM or vehicle for 1 h. Cells were then treated with CO-1 (1 µM, 10 min), washed and analysed by fluorescence confocal microscopy (scale bar: 10 µm). Lysotracker (75 nM) was used to stain lysosomes. Pearson's (P) and Manders' (M1 and M2) coefficients: **A**) RBM5-177-Lysotracker (P=0.725; M1=0.631; M2=0.760). **B**) RBM5-177-Lysotracker (P=0.754; M1=0.723; M2=0.713).

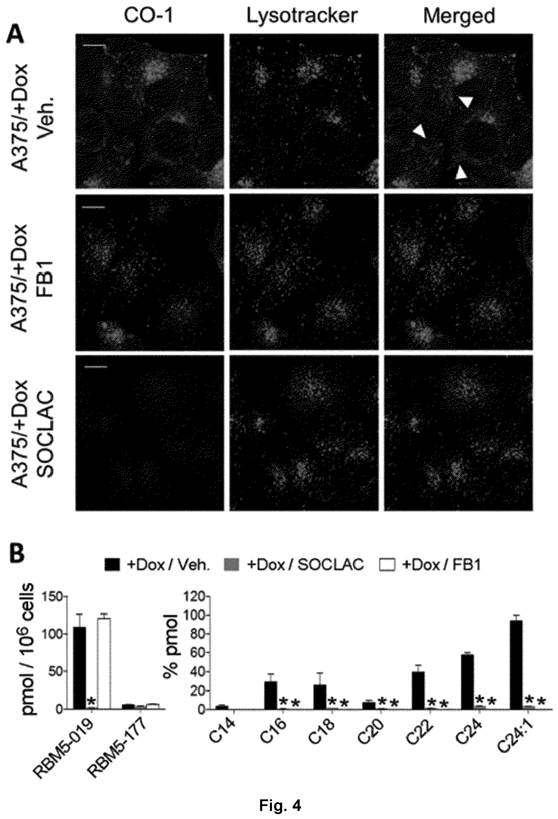

**Fig. 4****.** CerS activity-dependence of labeling with RBM5-177. A375 cells stimulated (+Dox) with for AC overexpression were incubated for with RBM5-177 4 h (5 µM, 4 h), prior treatment with vehicle, SOCLAC (5 µM, 1 h) or FB1 (100 µM, 24 h). The click reaction was carried out by incubation with CO-1 (1 µM ,10 min). Cells were analysed by fluorescence confocal microscopy (scale bar: 10 µm) (A) or the lipid extracts were analyzed by UPLC-HRMS (**B**). Lysotracker (75 nM) was used to stain lysosomes. White arrowheads point out putative mitochondria staining. Pearson's (P) and Manders' (M1 and M2) coefficients: RBM5-177-Lysotracker with vehicle (P=0.816; M1=0.791; M2=0.778) and RBM5-177-Lysotracker with FB1 (P=0.841; M1=0.717; M2=0.761). Data in B (mean ± SD) were obtained from two different experiments with triplicates. Data were analysed by one-way ANOVA followed by Dunnett's multiple comparison post-test if ANOVA P < 0.05 (*, P < 0.001 from vehicle). The right panel in B corresponds to the acyl chain of reacylated RBM5-019 (C14, tetradecanoyl, etc.).

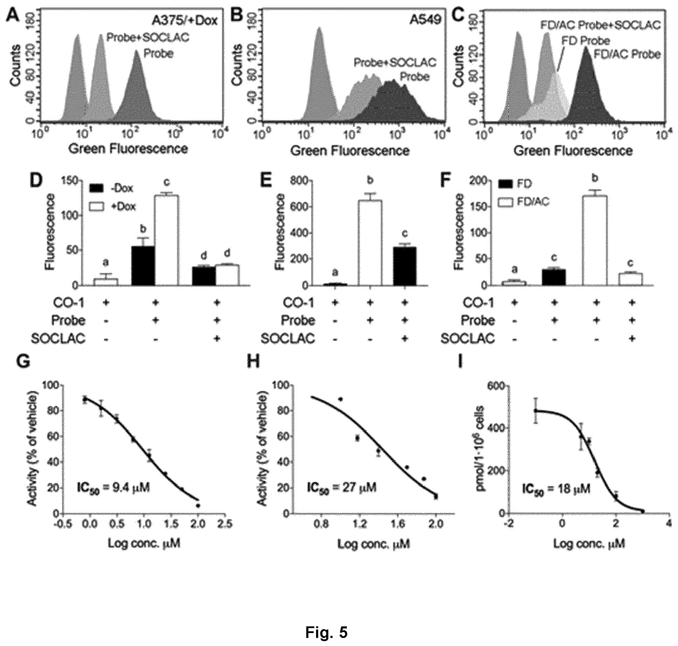

**Fig. 5****.** AC activity determination by flow cytometry with RBM5-177/CO-1. A375 cells stimulated (+Dox) or not (-Dox) with doxycycline for AC overexpression (**A,D**), A549 cells (**B,E**) or FD and FD/AC cells (**C,F**) were incubated with RBM5-177 (5 µM, 4 h), prior treatment with SOCLAC (5 µM, 1 h) or vehicle, and then with CO-1 (1 µM, 10 min). Cells were analyzed by flow cytometry measuring green fluorescence. Data (mean ± SD) were obtained from two or three different experiments with triplicates. Data were analysed by two-way (D) or one-way (E,F) ANOVA followed by Bonferroni's multiple comparison post-test if ANOVA P < 0.05. Different letters denote a statistical significance (P < 0.001). Representative histograms of panel D-E are given in panels A-C. G-I) A375/+Dox were incubated for 4h with different concentrations of DM120 (DM120 is the compound of formula: 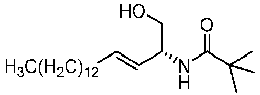 described in Bedia C, et al. Chem Phys Lipids 2008; 156: 33-40) in the presence of substrate ((G), RBM14C12 (40 µM) (RBM14C12 is the compound N-((2S,3R)-1,3-dihydroxy-5-((2-oxo-2H-chromen-7-yl)oxy)pentan-2-yl)dodecanamide, commercially available in Avanti polar lipids, Inc) (H) RBM5-177) or vehicle (I). AC activity was determined as reported (G) (C. Bedia, J Lipid Res 2010, 51, 3542-3547). by flow cytometry after labelling with CO-1 (1 µM, 10 min) (H) or by UPLC-HRMS quantification of the amounts of RBM5-019 (I). Data (mean ± SD) were obtained from two to three experiments with triplicates. Curve fitting with the sigmoidal dose-response (variable slope) equation afforded the IC₅₀ values detailed in the figure. A-F: Fluorescence at excitation /emission 488/525 nm.

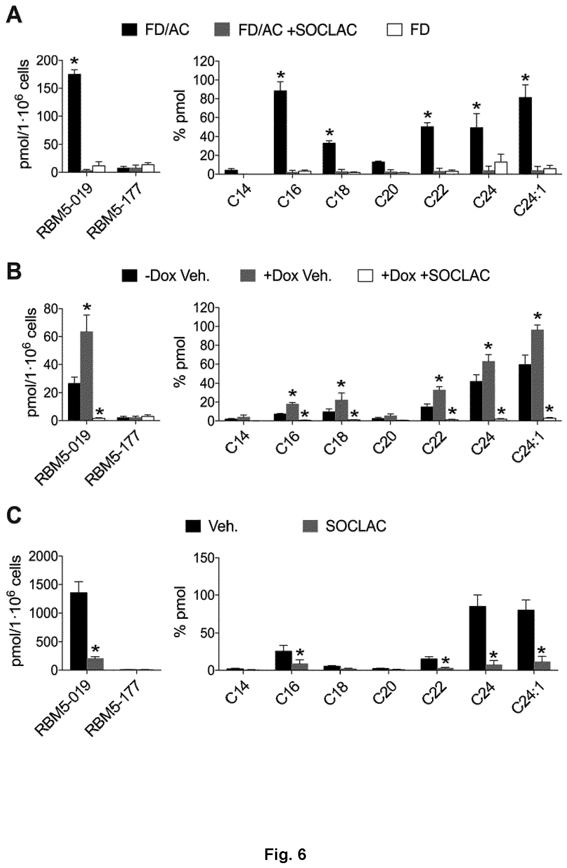

**Fig. 6****.** AC activity-dependence of RBM5-177 metabolization. FD and FD/AC cells (**A**), A375/+Dox or A375/-Dox (**B**) and A549 cells (**C**) were incubated with RBM5-177 (5 µM, 4 h), prior treatment with SOCLAC (5 µM, 1 h) or vehicle (Veh.) and the lipid extracts were analyzed by UPLC-HRMS. Data (mean ± SD) were obtained from three different experiments with triplicates. Data were analysed by two-way ANOVA test followed by Dunnett's multiple comparison test if ANOVA P < 0.05. (*, P < 0.001 from vehicle or FD cells). The right panels correspond to the acyl chain of reacylated RBM5-019 (C14, tetradecanoyl, etc.).

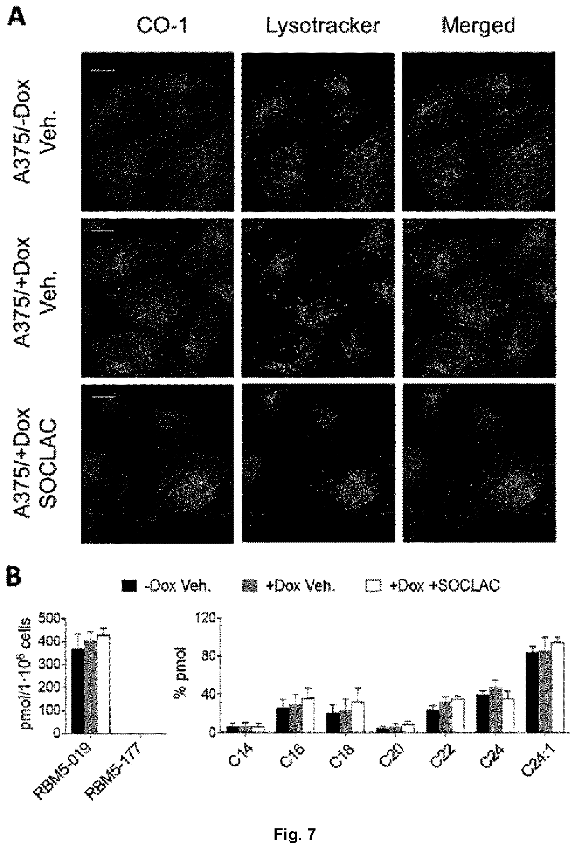

**Fig. 7****.** AC activity-independence of lysosome staining with RBM5-019. A375/+Dox) or A375/-Dox were incubated with RBM5-019 (5 µM, 20 min) prior treatment with SOCLAC (5 µM, 1 h) or vehicle (Veh.). The click reaction was carried out by incubation with CO-1 (10 min, 1 µM) and cells were analysed by fluorescence confocal microscopy (scale bar: 10 µm) (**A**) or the lipid extracts were analysed by UPLC-HRMS (**B**). Lysotracker (75 nM, 30 min) was used to stain lysosomes. Pearson's (P) and Manders' (M1 and M2) coefficients: RBM5-019-Lysotracker in A375/-Dox with vehicle (P=0.845; M1=0.829; M2=0.733), RBM5-019-Lysotracker in A375/+Dox with vehicle (P=0.869; M 1=0.836; M2=0.730) and RBM5-019-Lysotracker in A375/+Dox with SOCLAC (P=0.863; M1=0.810; M2=0.795). Data (mean ± SD) were obtained from three different experiments with triplicates. Data were analysed by two-way ANOVA test followed by Dunnett's multiple comparison post-test if ANOVA P < 0.05. The right panel in B corresponds to the acyl chain of reacylated RBM5-019 (C14, tetradecanoyl, etc.)

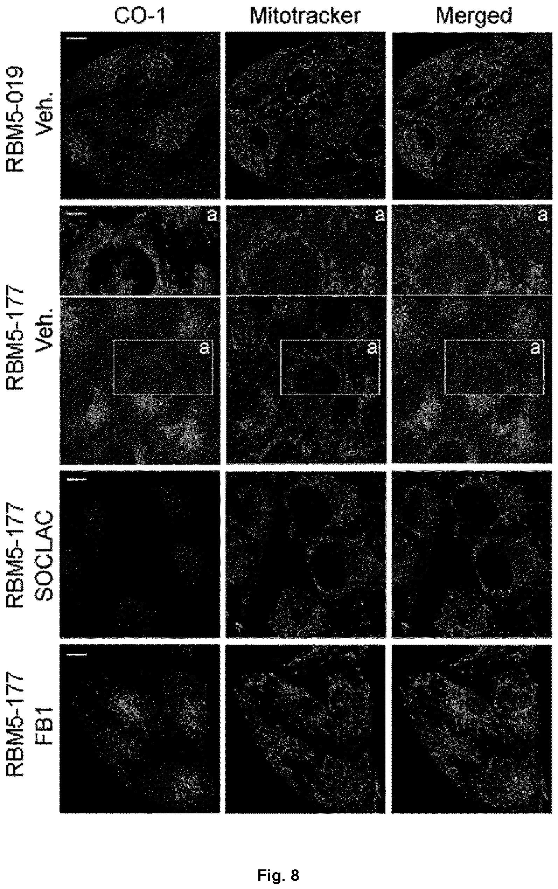

**Fig. 8****.** CerS activity-dependence of mitochondria staining with RBM5-177. AC overexpressing A375 cells(A375/+Dox) were incubated with the indicated probes (5 µM, 4 h) prior treatment with Soclac (5 µM, 1 h), FB1 (100 µM, 24 h) or vehicle (Veh.). The click reaction was carried out by incubation with CO-1 1 µM for 10 min. Mitotracker (75 nM) was used to stain mitochondria. Cells were analyzed by fluorescence confocal microscopy (scale bar: 10 µm). Mitochondria labelling is shown in the amplification of the selected area (a). Pearson's (P) and Manders' (M1 and M2) coefficients: RBM5-019-Mitotracker in A375/+Dox with vehicle (P=0.298; M1=0.179; M2=0.070), RBM5-177-Mitootracker in A375/+Dox with vehicle (P=0.355; M1=0.194; M2=0.697) and RBM5-177-Mitootracker in A375/+Doxwith FB1 (P=0.224; M1=0.059; M2=0.043).

### Examples

### Example 1: CHEMISTRY

Compounds of Formula (I) may be prepared following different methods known by a person skilled in the field of organic synthesis. In particular, they may be obtained following the route depicted in Scheme 1.

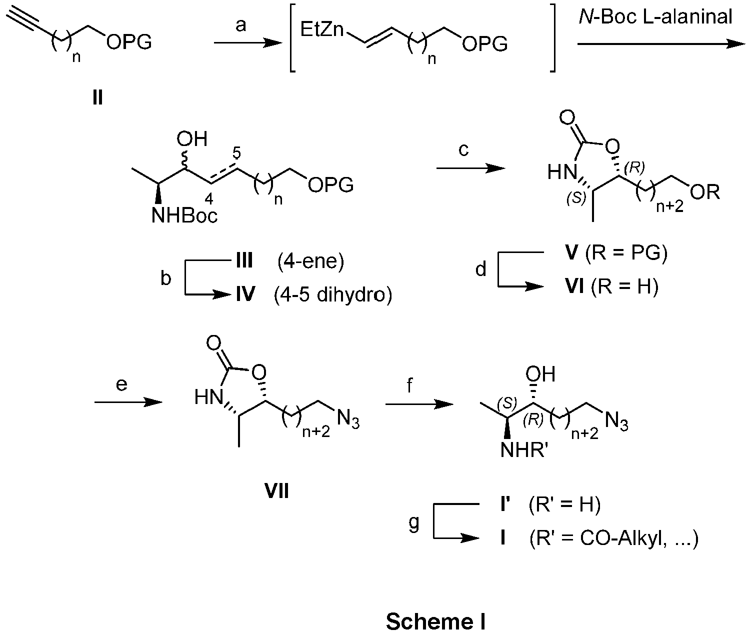

Therefore, after a process of hydrozirconation/transmetallation of alkyne II, followed by condensation with *N*-Boc alaninal compound **III** is afforded as an enriched inseparable mixture of diastereoisomers, which after hydrogenation gives a mixture of isomers **IV.** The major diastereomer is separated and an intramolecular *N*-Boc promoted is achieved to obtain oxazolidinones **V.** Deprotection, installation of the azide group and alkaline hydrolysis renders compounds type I'. Finally, the desired acyl compound **I** is obtained by acylation of the free amine group.

The inventors of the present invention have carried out some assays using the following compounds 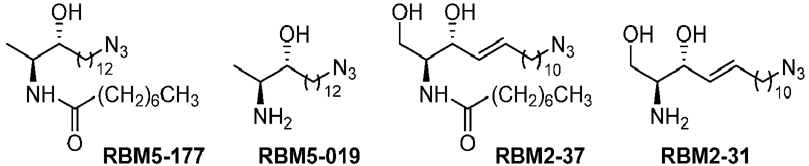 **and the BODIPY-labelled bicyclononyne CO-1 as fluorogenic reporter.**

Below, the experimental procedure of the steps in scheme 1 is explained in detail for RBM5-177 (compound type I).

### Reagents and general methods

All chemicals were purchased from commercial sources and used as received unless otherwise noted. Dry solvents (THF, DMF and DCM), obtained from a PureSolv dispenser and subsequently degassed with inert gas, were used in most reactions. Synthesis grade (Hexane, EtOAc, Et2O and DCM) or HPLC-grade (MeOH) solvents were used for extractions and purifications. Unless otherwise specified all reactions were performed under Ar inert atmosphere. Progression of the reactions was controlled by thin layer chromatography (TLC), using ALUGRAM^{®} SIL G/UV254 (Macherey-Nagel) silica gel pre-coated aluminum sheets (Layer: 0.2 mm, silica gel 60). Compounds were detected by using UV light (λ=254 nm) and a stain solution of phosphomolibdic acid (5.7% in EtOH). Compounds were purified by flash column chromatography, using silica gel (Chromatogel 60Å, 35-75 µm) as stationary phase. Mobile phases and gradients are specified in each case in the following section. ¹H and ¹³C Nuclear Magnetic Resonance spectra were recorded on a Varian - Mercury 400 (¹H NMR at 400 MHz and ¹³C NMR at 100.6 MHz) spectrometer using CDCl₃ or CD₃OD as solvent. Chemical shifts of deuterated solvents were used as internal standards. Chemical shifts are given in parts per million (ppm) and coupling constants (*J*) in Hertz (Hz). Fourier transform infrared spectra were recorded on a FTIR Avatar 360 spectrophotometer. Samples were recorded neat using an attenuated total reflectance (ATR) accessory, or as thin films on NaCl plates (specified in each case in the following section). IR frequencies are reported in wave number (cm⁻¹). Specific optical rotations were recorded on a digital Perkin-Elmer 34 polarimeter at 25 °C in a 1-dm 1-mL cell, using a sodium light lamp (λ=589 nm). Specific optical rotation values ([α]D) are expressed in deg⁻¹·Cm³·g⁻¹, and concentrations (c) are reported in g/100 mL of solvent. High-resolution mass spectra were recorded on a Waters Micromass LCT Premier apparatus equipped with a dual electrospray (ESI) LockSpray ion source. Data were acquired in positive ESI. Samples were analyzed by FIA (Flow Injection Analysis), using ACN/water (70:30) as mobile phase. Samples were analyzed using a 10 µL volume injection. M/z ratios are reported as values in atomic mass units.

### Intermediate II: tert-Butyl(dodec-11-yn-1-yloxy)diphenylsilane (RBM5-013)

Neat TBDPSCI (7.0 mL, 26.4 mmol) was added dropwise to an ice-cooled suspension of dodec-11-yn-ol (4.0 g, 22.0 mmol) and imidazole (3.0 g, 43.9 mmol) in anhydrous CH₂Cl₂ (80 mL). After stirring for 2 h at rt, the reaction was quenched by the addition of water (50 mL), and the resulting mixture was extracted with CH₂Cl₂ (3 x 50 mL). The combined organic extracts were washed with brine, dried over anhydrous MgS0₄, and concentrated under reduced pressure. The crude product was purified by flash column chromatography (from 0 to 2 % Et₂O in hexane) to give compound **RBM5-013** (7.8 g, 84 %) as a colourless oil.
¹ H NMR (400 MHz, CDCl₃) δ 7.69 - 7.65 (m, 4H), 7.44 - 7.35 (m, 6H), 3.65 (t, *J* = 6.5 Hz, 2H), 2.18 (td, *J* = 7.1, 2.6 Hz, 2H), 1.94 (t, *J* = 2.6 Hz, 1H), 1.61 - 1.46 (m, 4H), 1.36 (dt, *J* = 21.3, 7.6 Hz, 4H), 1.26 (s, 8H), 1.05 (s, 9H).
¹³C NMR (101 MHz, CDCl₃) δ 135.7, 134.3, 129.6, 127.7, 84.9, 68.2, 64.1, 32.7, 29.7, 29.6, 29.5, 29.2, 28.9, 28.6, 27.0, 25.9, 19.4, 18.5.
ATR-FTIR (neat) (v, cm⁻¹): 3311, 3071, 2930, 2856, 1471, 1428, 1389, 1361, 1111, 823, 739, 701, 687, 613.

### Intermediate III: tert-Butyl ((2S,E)-15-((tert-butyidiphenyisilyl)oxy)-3-hydroxypentadec-4-en-2-yl)carbamate (RBM5-014)

Neat **RBM5-013** (1.8 g, 4.2 mmol) was added to an ice-cooled suspension of Cp₂Zr(H)Cl (1.3 g, 5.1 mmol) in anhydrous CH₂Cl₂ (5 mL) under argon. After stirring at rt for 40 min, the resulting orange solution was cooled to -40 °C and then treated with Et₂Zn (1.0 M in hexanes, 5.7 mL, 5.7 mmol), followed by the dropwise addition of a solution of *N*-Boc L-alaninal (700 mg, 4.04 mmol) in anhydrous CH₂Cl₂ (3 mL). After stirring for 2 h at 0 °C, the reaction mixture was poured onto ice-water and extracted with CH₂Cl₂ (3 x 50 mL). The combined organic extracts were washed with brine, dried over anhydrous MgS0₄, filtered and concentrated *in vacuo.* Purification of the crude product by flash column chromatography (from 0 to 30 % EtOAc in hexanes) furnished **RBM5-014** (450 mg, 19 %, inseparable 5:1 mixture of *syn*/*anti* diastereomers) as a light-yellow thick oil.
¹H NMR (400 MHz, CDCl₃) (*syn* diastereomer) δ 7.69 - 7.65 (m, 4H), 7.44 - 7.35 (m, 6H), 5.71 (dtd, *J* = 15.4, 6.7, 1.0 Hz, 1H), 5.47 (ddt, *J* = 15.4, 7.1, 1.5 Hz, 1H), 4.63 (br s, 1H), 3.96 - 3.91 (m, 1H), 3.65 (app t, *J* = 6.5 Hz, 3H), 2.26 (s, 1H), 2.08 - 2.00 (m, 2H), 1.60 - 1.51 (m, 2H), 1.45 (s, 9H), 1.40 - 1.31 (m, 4H), 1.25 (t, *J* = 3.4 Hz, 10H), 1.14 (d, *J* = 6.8 Hz, 3H), 1.05 (s, 9H).
¹³C NMR (101 MHz, CDCl₃) (*syn* diastereomer) δ 156.2, 135.6, 134.2, 133.8, 129.6, 129.5, 127.6, 79.3, 75.8, 64.0, 51.0, 32.6, 32.4, 29.7, 29.6, 29.5, 29.4, 29.3, 29.2, 28.5, 26.9, 25.8, 19.2, 17.6.
FTIR (NaCl) (v, cm⁻¹): 3439, 3071, 2929, 2855, 1693, 1502, 1428, 1390, 1366, 1171, 1111, 1050, 702, 603.
HRMS calcd. for C₃₆H₅₈NO₄Si ([M+H]⁺): 596.4130, found: 596.4130.

### Intermediate IV: tert-Butyl ((2S)-15-((tert-butyldiphenylsilyl)oxy)-3-hydroxypentadecan-2-yl)carbamate (RBM5-015)

Compound **RBM5-015** (light yellow oil, 260 mg, 86 %, inseparable 5:1 mixture of *syn*/*anti* diastereomers) was obtained from alkene **RBM5-014** (300 mg, 0.50 mmol), and Rh/Al₂O₃ (30 mg) in degassed EtOAc (10 mL) by hydrogenation at 1 atm. After stirring for 3 h, the catalyst was removed by filtration through a Celite^{®} pad, and the solids were rinsed with EtOAc (3 x 10 mL). The combined filtrates were concentrated *in vacuo,* and the resulting residue was subjected to flash chromatography on silica gel. The title compound was purified by flash chromatography (from 0 to 18 % EtOAc in hexane).
¹H NMR (400 MHz, CDCl₃) (*syn* diastereomer) δ 7.69 - 7.65 (m, 4H), 7.44 - 7.35 (m, 6H), 4.66 (br s, 1H), 3.65 (app t, *J* = 6.5 Hz, 3H), 3.51 - 3.44 (m, 1H), 1.59 - 1.51 (m, 2H), 1.48-1.40 (m, 11H), 1.36 - 1.22 (m, 18H), 1.17 (d, *J* = 6.8 Hz, 3H), 1.05 (s, 9H).
¹³C NMR (101 MHz, CDCl₃) *(syn* diastereomer) δ 156.3, 135.7, 134.3, 129.6, 127.7, 79.4, 75.0, 64.1, 50.3, 34.3, 33.6, 32.7, 29.8, 29.8, 29.8, 29.7, 29.7, 29.5, 28.5, 28.5, 27.0, 26.2, 25.9, 25.8, 19.3, 18.5.
FTIR (NaCl) (v, cm⁻¹): 3438, 3071, 3051, 2927, 2854, 1715, 1687, 1501, 1365, 1167, 1105, 700.
HRMS calcd. for C₃₆H₆₀NO₄Si ([M+H]⁺): 598.4286, found: 598.4270.

### Intermediate V: (4S,5R)-5-(12-((tert-Butyldiphenylsilyl)oxy)dodecyl)-4-methyloxazolidin-2-one (RBM5-016)

Neat methanesulfonyl chloride (109 µL, 1.4 mmol) was added to an ice-cooled stirred solution of **RBM5-015** (560 mg, 0.9 mmol) in anhydrous CH₂Cl₂ (20 mL) containing Et₃N (260 µL, 1.9 mmol). After stirring at rt for 2 h, the reaction mixture was quenched by adding water (15 mL) and extracted with CH₂Cl₂ (3 x 25 mL). The combined organic extracts were washed with brine (25 mL), dried over anhydrous MgS0₄, and evaporated to dryness. The resulting solid residue was taken up in 1,2-DCE (20 mL), treated with Et₃N (653 µL, 4.7 mmol) and heated to reflux. After stirring overnight, the reaction was cooled to rt, water was added (15 mL), and the resulting mixture was extracted with CH₂Cl₂ (3 x 25 mL). The combined organic extracts were washed with brine (25 mL), dried over anhydrous MgS0₄, filtered and concentrated under reduced pressure. Flash column chromatography of the residue (from 0 to 30 % EtOAc in hexane) yielded **RBM5-016** (300 mg, major (4*S,*5*R*)*-anti*-isomer, 61 %), and the minor (4*S*,5*S*)-*syn*-isomer (80 mg, 16 %) as light-yellow oils. The configuration of compound **RBM5-016** was assigned by *nOe* experiments, similarly as reported by us in a previous related work (J. L. Abad et al. J. Org. Chem. 2013, 78, 5858-66).
[α]²⁰_{D} = +6.5 (c = 1, CHCl₃).
¹H NMR (400 MHz, CDCl₃) δ 7.69 - 7.65 (m, 4H), 7.44 - 7.34 (m, 6H), 4.88 (br s, 1H), 4.56 (ddd, *J* = 9.0, 7.1, 3.8 Hz, 1H), 3.88 (app p, *J* = 6.7 Hz, 1H), 3.65 (t, *J* = 6.5 Hz, 2H), 1.78 - 1.69 (m, 1H), 1.60 - 1.46 (m, 5H), 1.39 - 1.21 (m, 16H), 1.16 (d, *J* = 6.5 Hz, 3H), 1.04 (s, 9H).
¹³C NMR (101 MHz, CDCl₃) δ 160.0, 135.6, 134.3, 129.5, 127.6, 80.3, 64.1, 51.2, 32.7, 29.7, 29.7, 29.7, 29.6, 29.5, 29.5, 29.5, 29.2, 27.0, 26.0, 25.9, 19.3, 15.9. FTIR (NaCl) (v, cm⁻¹): 3583, 3283, 3071, 2928, 2855, 1754, 1428, 1110, 702.
HRMS calcd. for C₃₂H₅₀NO₃Si ([M+H]⁺): 524.3554, found: 524.3544.

### Intermediate VII: (4S,5R)-5-(12-Azidododecyl)-4-methyloxazolidin-2-one (RBM5-018)

Step 1: A solution of **RBM5-016** (220 mg, 0.4 mmol) in anhydrous THF (10 mL) was treated with TBAF (1.0 M in THF, 840 µL, 0.8 mmol). After stirring for 2 h at 0 °C, the reaction was quenched by adding saturated aqueous NH₄Cl (10 mL) and extracted with Et₂O (3 x 25 mL). The combined organic extracts were washed with brine (2 x 10 mL), dried over anhydrous MgSO₄, concentrated under reduced pressure, and purified by flash column chromatography (from 0 to 100 % EtOAc in hexanes) to give the deprotected alcohol **RBM5-017** (110 mg, 92 %) as an off-white solid.
[α]²⁰_{D} = +12.3 (c = 1, CHCl₃).
¹ H NMR (400 MHz, CDCl₃) δ 6.09 (br s, 1H), 4.53 (td, *J* = 9.3, 8.4, 3.5 Hz, 1H), 3.87 (app p, *J* = 6.7 Hz, 1H), 3.61 (t, *J* = 6.7 Hz, 2H), 1.87 (br s, 1H), 1.77 - 1.64 (m, 1H), 1.59 - 1.43 (m, 3H), 1.37 - 1.19 (m, 18H), 1.13 (d, *J* = 6.5 Hz, 3H).
¹³C NMR (101 MHz, CDCl₃) δ 159.9, 80.4, 63.1, 51.2, 32.9, 29.7, 29.6, 29.6, 29.5, 29.5, 29.5, 29.5, 29.2, 25.9, 25.8, 16.0.
ATR-FTIR (neat) (v, cm⁻¹): 3421, 3282, 2980, 2920, 2849, 1732, 1698, 1392, 1235, 1105.
HRMS calcd. for C₁₆H₃₂NO₃ ([M+H]⁺): 286.2377, found: 286.2360.

Step 2: A solution of *N*-bromosuccinimide (49 mg, 0.2 mmol) in DMF (1 mL) was added dropwise to an ice-cooled solution of **RBM5-017** (65 mg, 0.2 mmol) and triphenylphosphine (66 mg, 0.3 mmol) in DMF (2 mL). After stirring at rt for 2 h, NaN₃ (44 mg, 0.7 mmol) was added in one portion and the mixture was heated to 75 °C and stirred for additional 5 h. After completion (TLC), the reaction was diluted with brine (10 mL) and the resulting suspension was extracted with Et₂O (3 x 20 mL). The combined organic extracts were thoroughly washed with brine (3 x 5 mL), dried over anhydrous MgS0₄, concentrated to dryness and the residue was purified by flash column chromatography (from 0 to 34 % EtOAc in hexanes) to afford **RBM5-018** (50 mg, 71 %) as an off-white solid.
[α]²⁰_{D} = +12.3 (*c* = 1, CHCl₃).
¹H NMR (400 MHz, CDCl₃) δ 5.89 (br s, 1H), 4.54 (ddd, *J* = 9.5, 7.4, 3.9 Hz, 1H), 3.88 (app p, *J* = 6.7 Hz, 1H), 3.24 (t, *J* = 7.0 Hz, 2H), 1.76 - 1.67 (m, 1H), 1.62 - 1.54 (m, 2H), 1.54 - 1.44 (m, 2H), 1.39 - 1.21 (m, 17H), 1.14 (d, *J* = 6.5 Hz, 3H).
¹³C NMR (101 MHz, CDCl₃) δ 159.9, 80.3, 51.6, 51.2, 29.6, 29.6, 29.6, 29.5, 29.5, 29.5, 29.2, 29.2, 28.9, 26.8, 26.0, 16.0.
ATR-FTIR (neat) (v, cm⁻¹): 3273, 2981, 2917, 2850, 2093, 1742, 1712, 1470, 1398, 1253.
HRMS calcd. for C₁₆H₃₁N₄O₂ ([M+H]⁺): 311.2442, found: 311.2432.

### Compound type I': (2S,3R)-2-Amino-15-azidopentadecan-3-ol (RBM5-019)

Compound **RBM5-018** (55 mg, 0.18 mmol) was dissolved in EtOH-2.0 N aq. NaOH (1:1) (6 mL) and the resulting solution was heated to reflux temperature. After stirring for 4 h, the reaction mixture was concentrated to dryness and the residue was diluted with water (5 mL) and extracted with EtOAc (3 x 20 mL). The combined organic extracts were dried over anhydrous MgS0₄, evaporated, and the crude product was purified by flash column chromatography (from 0 to 20 % MeOH in CH₂Cl₂) to furnish **RBM5-019** (40 mg, 79 %) as an off-white waxy solid.
[α]²⁰_{D} = +5.2 (c = 1, CHCl₃).
¹H NMR (400 MHz, CDCl₃) δ 3.42 (ddd, *J* = 8.0, 4.7, 3.5 Hz, 1H), 3.24 (t, *J* = 7.0 Hz, 2H), 2.96 (qd, *J* = 6.5, 3.5 Hz, 1H), 1.76 - 1.64 (m, 3H), 1.63 - 1.55 (m, 2H), 1.39 - 1.22 (m, 20H), 1.00 (d, *J* = 6.6 Hz, 2H).
¹³C NMR (101 MHz, CDCl₃) δ 74.9, 51.6, 50.5, 32.6, 29.9, 29.7, 29.7, 29.7, 29.7, 29.6, 29.3, 29.0, 26.9, 26.4, 17.0.
ATR-FTIR (neat) (v, cm⁻¹): 3336, 2919, 2850, 2094, 1581, 1467, 1371, 1292.
HRMS calcd. for C₁₅H₃₃N₄O ([M+H]⁺): 285.2649, found: 285.2640.

### Compound type I: N-((2S,3R)-15-azido-3-hydroxypentadecan-2-yl)octanamide (RBM5-177)

To a solution of 1-Ethyl-3-(3-dimethylaminopropyl)carbodiimide hydrochloride (EDC.HCl) (14 mg, 70 µmol) and HOBt (7 mg, 52 µmol) in anhydrous CH₂Cl₂ (2.5 mL) was added octanoic acid (7 mg, 48 µmol) under argon atmosphere. The resulting mixture was vigorously stirred at rt for 10 min, and next added dropwise to a solution of **RBM5-019** (14 mg, 44 µmol) and Et₃N (30 µL, 0.2 mmol) in anhydrous CH₂Cl₂ (2.5 mL). The reaction mixture was stirred at rt for 2 h under argon atmosphere. Subsequently, the mixture was diluted by addition of CH₂Cl₂ (10 mL) and washed with brine (2 x 5 mL). The organic layer was dried over anhydrous MgS0₄, filtered and the solvent was removed under reduced pressure. Purification of the crude by flash chromatography (from 0 to 5 % MeOH in CH₂Cl₂) gave the pure amide **RBM5-177** (16 mg, 89 %) as an off-white waxy solid.

¹H NMR (400 MHz, CDCl₃) δ 5.86 (s, 1H), 4.01 (s, 1H), 3.62 (s, 1H), 3.25 (t, J = 6.9 Hz, 2H), 2.31 (s, 1H), 2.17 (t, J = 7.0 Hz, 2H), 1.67 - 1.54 (m, 4H), 1.43 - 1.21 (m, 28H), 1.09 (d, J = 6.6 Hz, 3H), 0.91 - 0.82 (m, 3H).
¹³C NMR (101 MHz, CDCl₃) δ 173.5, 74.5, 51.6, 49.7, 37.0, 33.7, 31.8, 29.8, 29.7, 29.7, 29.6, 29.6, 29.4, 29.3, 29.1, 29.0, 26.8, 26.1, 26.0, 22.7, 14.2, 14.2.
HRMS calcd. for C₂₃H₄₇N₄O₂ ([M+H]⁺): 411.3694, found: 411.3699.

Compounds RBM2-37 and RBM2-31 are not within the scope of the invention and are provided only as comparative compounds.
The synthesis of compounds RBM2-031, RBM2-037 and CO-1 has been previously reported in the literature (S. H. Alamudi, et al., Nat. Commun. 2016, 7, 11964 and M. Garrido, et al. ChemBioChem 2015, 16, 641-650).

### Example 2: BIOLOGICAL STUDIES

### Deoxydihydroceramides as ceramidases substrates

RBM5-177 underwent an almost complete metabolization to both the free base (RBM5-19) and differently transacylated products in A549 lung adenocarcinoma cells (5 µM, 30 min) as concluded by UPLC-HRMS lipid analysis (Figure 1A). These results suggested that deoxydihydroceramides are hydrolyzed by at least one ceramidase.

To confirm this hypothesis, human recombinant neutral ceramidase (NC) or lysates of acid ceramidase (AC) and microsomes of alkaline ceramidases 1, 2 and 3 (ACER1, ACER2 and ACER3) from cells overexpressing these 4 enzymes were incubated with RBM5-177 and the RBM5-19 formation was quantified by UPLC-HRMS (ultra-high performance liquid chromatography-high resolution mass spectrometry). As shown in Figure 1B, RBM5-177 was transformed into the free amine only by AC. That AC was the enzyme responsible for the hydrolysis and no other acid amidase was involved was confirmed by incubation with SOCLAC, a specific irreversible AC inhibitor, which totally prevented the formation of RBM5-19 from RBM5-177 (Figure 1C).

### Monitoring of AC activity in intact cells by flow cytometry

Since RBM5-177 is specifically hydrolysed by AC, this probe could enable the unprecedented specific monitoring of AC activity in intact cells by flow cytometry prior to click reaction with the BODIPY labelled bicyclononyne CO-1 as reagent.
To validate this putative approach, the AC-activity dependence of the subcellular staining distribution by the RBM5-177/CO-1 system was first examined by fluorescence confocal microscopy. Thus, A549 cells, previously found to contain AC activity, were pulsed with RBM5-177 (5 µM, 30 min) and then the click reaction with CO-1 (1 µM) was carried out (30 min). As shown in Figure 2A.
Fluorescence confocal microscopy revealed that the labelling from RBM5-177/CO-1 co-localized with lysotracker (Commercial available marker from Thermofisher), indicating that the probe was directed to the lysosome in agreement with the lysosomal localization of AC. To verify that the RBM5-177/CO-1 labelling system was reliable, the experiment was performed in parallel with the canonical ceramide sibling RBM2-37. In accordance with the use of fluorescent ceramides as Golgi trackers, Golgi staining was observed with the RBM2-37/CO-1 staining system (Figure 2A). Importantly, when a 5h incubation step in fresh culture medium (Dulbecco's Modification of Eagle's Medium, DMEM) was introduced after the RBM5-177 pulse prior to CO-1 addition, staining remained stable with high co-localization with Lysotracker (Figure 2B). In contrast, the co-localization of the RBM2-37/CO-1 staining with the Golgi tracker decreased considerably and was found in other compartments (Figure 2B), in agreement with the probe metabolization as a ceramide analog.
These results supported the metabolic stability of RBM5-177. A similar outcome was found with the free base, RBM5-19, which largely remained in the lysosome after the 5 h chase (compare Figure 2C with Figure 2D) while labelling intensity from the canonical base, RBM2-31, which can be further metabolized via the ceramide catabolic pathway, decreased considerably (compare Figure 2C with Figure 2D). Besides confirming the metabolic stability of RBM5-19, these results also suggested that the AC-generated RBM5-19, which would be protonated in the acidic lysosomal pH, is largely retained inside the lysosome, as reported for other lipophilic amino alcohols such as propranolol, while sphingosine egress from the lysosome is not so restricted.

### Acid ceramidase activity in Farber cell lines, melanoma cells and control cells

The above results suggested that the *in situ* generated RBM5-19 might be responsible for the observed lysosome staining. In order to prove this possibility, the effect of AC overexpression and AC activity deficiency/blockade on lysosome labelling by RBM5-177 was analysed using Farber disease fibroblasts lacking AC (FD) and the same cells transduced to overexpress AC (FD/AC), and AC-overexpressing melanoma A375 cells (A375/+Dox) and their wild type (WT) (A375/ Dox).
As depicted in Figure 3A, lysosome staining was observed in FD/AC cells after RBM5-177/CO-1 treatment, while no lysosomal labelling occurred in either FD cells or in SOCLAC pretreated FD/AC cells. In agreement, UPLC/HRMS showed the presence of RBM5-19 and reacylated products in FD/AC cells, but not in FD and SOCLAC pretreated FD/AC cells (Figure 6A), thus correlating the lysosome staining with AC activity. Furthermore, lysosome labelling intensity was higher in A375/+Dox than in A375/ Dox cells and the organelle staining in the latter was completely abolished by pre incubation with SOCLAC (Figure 3B). In agreement, UPLC-HRMS analysis of lipid extracts from cells at the same experimental conditions showed that levels of RBM5-19 and reacylated products were significantly higher in AC/+Dox than in AC/ Doc cells, while they dropped dramatically in the former upon SOCLAC pre treatment (Figure 6B). In contrast, lysosome labelling with RBM5-19 was not sensitive to AC overexpression or blockade (Figure 7A) with very high co-localization with lysosomes in all cases. Accordingly, levels of RBM5-19 and transacylated products were not affected either by differences in AC-activity (Figure 7B). These results indicated that RBM5-19 and not RBM5-177 was the responsible molecule for lysosome labelling, and that RBM5-177 labelling was AC-activity dependent.
Interestingly, in both AC overexpressing cells (FD/AC and A375/+Dox), incubation with RBM5-177 produced labelling also outside the lysosomes (Figure 3), which might be indicative of the extralysosomal CerS mediated re-acylation of the RBM5-19 fraction released from the acidic compartment. In order to confirm this hypothesis, the effect of the CerS inhibitor FB1 (from Sigma-Aldrich) on the intracellular stain distribution was tested. As shown in Figure 4A, the extralysosomal staining found in RMB5-019/CO-1 treated A375/+Dox cells was prevented by pre-incubation with FB1 (100 µM, 24 h), while the lysosomes labelling remained intact. UPLC-HRMS lipid analysis confirmed that transacylation was blocked with FB1, since no N-acylderivatives of RBM5-177 were detected and RBM5-19 levels remained unaltered (Figure 4B).

That the extralysosomal staining was due to the *in situ* generated base re acylation was further confirmed by treatment with SOCLAC, which avoided the staining of both lysosomes and extralysosomal structures (Figure 4A) and precluded the formation of both RBM5-19 and transacylated products, as concluded from their absence in the lipid extracts from SOCLAC pre treated/RBM5-177 treated cells (UPLC-HRMS)
(Figure 4B). Therefore, all these results proved that lysosome staining was due to RBM5-19 accumulation while the re-acylated products were responsible for the labelling outside lysosomes, which was shown to be CerS dependent. Importantly, these results also confirmed that not only hydrolysis, but also the formation of RBM5-177 transacylation products were dependent on the cell's AC activity.

After establishing that both lysosome and extralysosome staining with RBM5-177 were dependent on AC activity and that the staining was robust and long lasting, the unprecedented flow cytometry-based monitoring of AC activity was investigated. As shown in Figure 5, the number of cells labelled by RBM5-177/CO-1 highly correlated with both the confocal microscopy and the UPLC/HRMS data mentioned above. Thus, in A375 cells (Figure 5A and 5D) a significantly higher number of fluorescent counts was observed in AC-overexpressing than in non-induced cells and the signal significantly decreased with SOCLAC pre-incubation. Moreover, the effect of SOCLAC at significantly decreasing the number of labelled cells was also confirmed in the A549 cell line (Figure 5B and 5E), consistent with the absence of RBM5-19 and re-acylated products in the lipid extracts (Figure 6C). In the case of the two Farber cell models, the AC deficient FD cells were negligibly labelled with RBM5-177/CO-1, while a significant 7-fold increase in the number of fluorescent counts over FD were measured in the AC-transduced counterparts. In the latter, the number of labelled cells were reduced down to FD levels upon SOCLAC preincubation (Figure 5C and 5C).

Finally, the applicability of the flow cytometry method to test for AC-inhibitory activity was examined. Although its usefulness was already inferred from the results found with SOCLAC, we expanded the study to a reversible inhibitor, using the previously reported compound DM120 (C. Bedia, et al. Chem. Phys. Lipids 2008, 156, 33-40) in AC overexpressing A375 cells. First, DM120 AC inhibitory activity was examined using a fluorogenic assay (C. Bedia, et al. J Lipid Res 2010, 51, 3542-3547), which afforded an IC50 value of 9.4 µM (Figure 5G) without toxicity in the whole range of concentrations examined.

Using RBM5-177 as substrate and flow cytometry analysis after cell labelling with CO-1, the concentration-response curve yielded an IC50 of 27 µM (Figure 5H). A similar value (IC50 = 18 uM) was obtained by measuring the decrease in RBM5-19 amounts as DM120 concentration increased by UPLC-HRMS (Figure 5I). The slightly lower IC50 value obtained with the fluorogenic assay can be explained considering that while RBM5-177 is AC specific, the fluorogenic substrate is also accepted by neutral and alkaline ceramidase 3. Therefore, putative inhibition of these ceramidases by DM120 (its specificity for the different ceramidases has not been investigated) might contribute to the inhibition when monitored with the fluorogenic substrate.

Overall, these results demonstrate that RBM5-177 is a good probe for the flow cytometry monitoring of AC activity in intact cells using bicyclononynes such as CO-1 as click partners for labelling.

In all the conditions tested, the RBM5-19/CO-1 labeling system studied here exhibited exclusive localization in the lysosome with no mitochondria staining as found in co-localization studies with Mitotracker (Figure 8).
Interestingly, part of the RBM5-019 /CO-1 extralysosomal staining observed in AC-overexpressing cells (see Figure 4) was found to localize significantly with mitotracker (Mander's coefficient M2= 0.697) (Figure 8), although most of the labelling was found in the lysosome (Pearson's coefficient with mitotracker was 0.355). As expected, mitochondria labelling was precluded by independent treatments with FB1 and SOCLAC, proving that it was both AC and CerS-dependent.

### Conclusion

In summary, we have reported a simple, fast, robust and sensitive novel assay for the specific determination of AC activity in intact cells by flow-cytometry. The main advantage of this method over the fluorogenic assay is the probe specificity for AC, allowing the reliable determination of AC activity in intact cells. Conversely, the fluorogenic AC substrates are also hydrolyzed by NC and ACER3 and, although discrimination is achieved in cell-free systems by pH modulation, the specific measurement of AC activity in intact cells is not possible. Furthermore, the AC-dependent lysosome staining reported here can be optimized to be faster than the fluorogenic assay (30 min *vs* ≥3 h) and the readout is very persistent. On the other hand, the possibility of using RBM5-177 with CO-1 analogs with varying emission colors denotes an advantage over using the recently reported AC activity-based probe.
Furthermore, using cells with a high degree of AC expression, this system may be used to follow up the traffic of deoxysphingolipids, which can be of relevance in the study of conditions linked to altered deoxysphingolipid metabolism. Finally, the use of the RBM5-19/CO-1 system allows the robust and long lasting AC activity-independent lysosome staining in live cells.

Below, the experimental procedure of these biological studies is explained in detail:

### Materials

Dulbecco's modified Eagle's medium (DMEM), trypsin-EDTA, fetal bovine serum, doxycycline and tetracycline were purchased from Sigma. Zeocin was from Genaxxon Bioscience and blasticidin from CalBiochem. Recombinant human NC was obtained from R&D Systems. Hygromycin B, LysoTracker Red DND-99, Cell Light Golgi-RFP BacMan 2.0 and Mitotracker were from Thermo Scientific. Fumonisin B1 was purchased from Enzo. DM120 (C. Bedia et al. Chem. Phys. Lipids 2008, 156, 33-40) and CO-1 (S. H. Alamudi et al., Nat. Commun. 2016, 7, 11964) were synthesized as reported

### Cells

The A375 cell line stably overexpressing ASAH1 under the control of a doxycycline-responsive promoter was kindly provided by Dr. Carmen Bedia and Prof. Thierry Levade (Laboratoire de Biochimie Métabolique, Institut Fédératif de Biologie, CHU Purpan, INSERM UMR1037 CRCT, Toulouse, France) (). The antibiotic selection of this cell line was performed with blasticidin (3 µg/mL) and hygromycin B (250 µg/mL). Ectopic expression of AC was induced with doxycycline at 1 µg/mL for 24 h before use. Farber disease fibroblasts FD and FD/AC cell lines were a kind gift from Prof. Thierry Levade (Laboratoire de Biochimie Métabolique, CHU Toulouse, France). HeLa T-Rex ACER1-TET-ON and HeLa T-Rex ACER2-TET-ON cells stably overexpress ACER1 or ACER2 respectively under the control of a tetracycline-responsive promoter were obtanained from Prof. Cungui Mao (Department of Medicine, State University of New York, Stony Brook, NY, USA). The antibiotic selection of HeLa T-Rex TET-ON cells was performed with zeocin (25 µg/ml) and blasticidin (5 µg/ml). Expression of ACER1 or ACER2 was induced with tetracycline 0.05 µM for 24 h before use. MEF5 cell line was a kind gift of Prof. Richard Proia (Genetics of Development and Disease Branch, NIDDK, National Institutes of Health, Bethesda, MD, USA). It was chosen to study ACER3 because was defective in the NC gene and have high levels of ACER3 transcript. All cells were grown in a humidified 5% CO₂ atmosphere at 37 °C in DMEM medium supplemented with 10% fetal bovine serum.

### Cell lysates

Cells were suspended in the appropriate volume of a 0.25 M saccharose solution with the proteases inhibitors aprotinin (1 mg/ml), leupeptin (1 mg/mml) and PMSF (100 mM). The suspension was submitted to three cycles of a 5 s sonication (probe) at 10 watts/5 s resting on ice. The cell lysate was centrifuged at 600 g for 5 min. The supernatant was collected and protein concentration was determined as specified below.

### Microsomal preparations

Cell lysates obtained from HeLa T-Rex ACER1-TET-ON, HeLa T-Rex ACER2-TET-ON and MEF5 overexpressing ACER3 cells were transferred to ultracentrifuge tubes and were spun at 100000 g for 1 h at 4°C. Pellets were resuspended in a 0.25 M saccharose solution and protein concentration was determined as specified below.

### Determination of protein concentration

Protein concentrations were determined with BSA as a standard using a BCA protein determination kit (Thermo Scientific) according to the manufacturer's instructions.

### Cell viability

A375 cells were seeded at 2.5 × 10⁴ cells/mL in 96-well plates (0.1 ml/well) and grown for 24 h. Overexpression of AC was induced with doxycycline at 1 µg/mL for 24 h. Cell viability was examined in triplicate samples by MTT method after treatment with DM120 at different concentrations or with the corresponding percentage of vehicle for 4 h.

### Fluorogenic AC activity in intact cells

To determine AC activity in intact cells, A375 cells were seeded at 2.5 × 10⁴ cells/mL in 96-well plates (0.1 ml/well) and grown for 24 h. Overexpression of AC was induced with doxycycline at 1 µg/mL for 24 h. Medium was replaced by DM120 at different concentrations and substrate RBM14C12 at a final concentration of 40 µM in DMEM. The plate was incubated for 4 h at 37°C in 5% CO₂. The reaction was stopped with 25 µL of MeOH and then 100 µL of NalO₄ (2.5 mg/mL in glycine-NaOH buffer, pH 10.6) was added. After incubation at 37°C for 1 h, 100 µL of 0.1 M glycine-NaOH buffer (pH 10.6) was added and fluorescence was measured spectrophotometrically at excitation and emission wavelengths of 355 and 460 nm, respectively. The same reaction mixture without cells was used as blank.

### UPLC-HRMS

Cells were seeded at 5 × 10⁵ cells/mL into 6 well plates (1 mL/well) and were allowed to adhere for 24 h. Medium was replaced with fresh medium containing RBM5-177, RBM5-019 or RBM2-37 (5 µM final concentration). After 30 min, medium was removed; cells were washed with 400 µL PBS and harvested with 400 µL Trypsin-EDTA and 600 µL of medium. NC recombinant enzyme, lysates from A375-AC overexpressing cells, MEF5 or microsomes from HeLa T-Rex ACER1-TET-ON and HeLa T-Rex ACER2-TET-ON cells stably overexpress ACER1 orACER2 respectively were incubated with RBM5-177 or RBM5-019 (10 µM final concentration) for 3 h in the corresponding ceramidase activity buffer. Cell pellets were resuspended with 100 µL of H₂O and mixed with 750 µL of methanol: chloroform, 2:1. Samples were heated at 48°C overnight and next day, 75 µL of 1 M KOH in methanol were added, followed by 2 h incubation at 37°C. Afterwards, the saponification was neutralised with 75 µL of 1 M acetic acid and solvent was removed using a Speed Vac Savant SPD131 DDA (Thermo Scientific). Sphingolipid extracts, fortified with internal standards (N-dodecanoylsphingosine, *N*-dodecanoylglucosylsphingosine, *N-*dodecanoylsphingosylphosphorylcoline and C17-sphinganine 0.2 nmol each) were solubilised in 150 µL of methanol. Samples were then centrifuged at 9,300 g for 3 min and 130 µL of the supernatant were injected to a Waters Aquity UPLC system connected to a Waters LCT Premier Orthogonal Accelerated Time of Flight Mass Spectrometer (Waters,Milford, MA, USA) operated in positive electrospray ionisation mode. Full scan spectra from 50 to 1500 Da were acquired and individual spectra were summed to produce data points each 0.2 s. Mass accuracy and reproducibility were maintained by using an independent reference spray by the LockSpray interference. The analytical column was a 100 mm x2.1 mm i.d., 1.7 µm C8 Acquity UPLC BEH (Waters). The two mobile phases were phase A: methanol/water/formic acid (74/25/1 v/v/v); phase B: methanol/formic acid (99/1 v/v), both also contained 5 mM ammonium formate. A linear gradient was programmed-0.0 min: 80 % B; 3 min: 90 % B; 6 min: 90 % B; 15 min: 99 % B; 18 min: 99 % B; 20 min: 80 % B. The flow rate was 0.3 mL min-1.

### Confocal visualization

Cells were seeded at 1 × 10⁵ cells/mL on glass-bottom plates and allowed to adhere for 24 h. For golgi staining, cells were incubated with 40 µL/well of CellLight Golgi-RFP BacMam 2.0 for 24 h. Medium was replaced with fresh medium containing RBM5-177, RBM5-019, RBM2-37 or RBM2-31 (5 µM final concentration) and cells were incubated for 20 min at 37 °C. After this time, the medium was replaced with fresh medium containing CO-1 (1 µM final concentration), and cells were incubated for 10 min. Then cells were washed 3 times with DMEM for 5 min at 37 °C, 75 nM LysoTracker Red DND-99 or 75 nM Mitotracker in phenol red-free DMEM was added to stain lysosomes or mitochondria, and cells were kept in the incubator for microscope analysis. Staining was viewed with a Zeiss LSM 880 confocal microscope or Leica TCS SP8-Hyvolution. Image analysis was performed using Fiji software.^{[7]} To measure the Manders's and Pearson's coefficients a threshold including organelles and discarding the background was set. Co-localization coefficients were measured using the JaCoP plugin (S. Bolte et al. J. Microsc. 2006, 224, 213-232) on the different stacks of images (at least n=4) with each stack containing 3-10 cells.

### AC activity by flow cytometry

Cells were seeded at 1 × 10⁵ cells/mL into 6 well plates (1 mL/well) and were allowed to adhere for 24 h. Medium was replaced with fresh medium containing Soclac (5 µM final concentration) and incubated for 1 h.
After this time, the medium was replaced with RBM5-177, RBM5-019 or RBM2-37 (5 µM final concentration) +/- Soclac and incubated for 20 min. In the experiments with DM120, the compound, Soclac and RBM5-177 were incubated together for 4 h. After probes incubation, the medium was replaced with fresh medium containing CO-1 (1 µM final concentration), and cells were incubated for 10 min. Then cells were washed 3 times with DMEM for 5 min at 37 °C, and finally washed with 400 µL PBS and harvested with 400 µL Trypsin-EDTA and 600 µL of medium. Pellets were resuspended with 200 µL of PBS and stained cells were analyzed by using a Guava EasyCyte^{™} flow cytometer (Merck Millipore, Billerica, MA) measuring green fluorescence (488/525 nm). Data analysis was performed using the Multicycle AV program (Phoenix Flow Systems, San Diego, CA).

### Statistical analysis

All experiments were performed in duplicate or triplicate and data from at least two independent experiments are given as means ± SD. For comparison between means, data were analysed by one-way ANOVA followed by Dunnett's (pairwise comparisons of multiple groups with a single control group) or Bonferroni's (comparisons between all data groups) multiple comparison post-tests. Differences with P < 0.05 were considered significant. Dose response curves were adjusted with sigmoidal dose-response curve with variable slope equation. All tests were run with the GraphPad software. The confocal images are representative of at least two independent experiments with a similar pattern.

## Claims

1. A compound of formula (I) 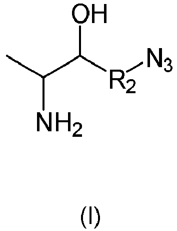 **characterized in that**:
R₁ is -CO-(C3-C20) alkyl, -CO-(C3-C20) alkenyl, -CO-(C3-C20) alkynyl, or CO-R₃-Y-
R₄, where Y is selected from S, O, NH, SO, SO₂, CO, NHCO, CONH, COO and OCO,
R₃ is (C1-Cₐ) alkylene, (C2-Cₐ) alkenylene or (C2-Cₐ) alkynylene,
R₄ is (C1-C_{b}) alkyl, (C2-C_{b}) alkenyl or (C2-C_{b}) alkynyl, being a+b≤20, and both
a and b different from zero,
R₂ is a C4-C18 linear alkylene, C4-C18 linear alkenylene or a C4-C18 linear alkynylene.

2. The compound according to claim 1 wherein the compound of formula (I) is the steroisomer:

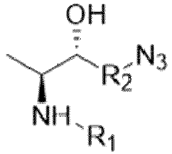

3. The compound according to claim 1 or 2 wherein R₁ is -CO-(C3-C20) alkyl

4. The compound according to any of claims 1 to 3 wherein R₂ is a C4-C18 linear alkylene.

5. A method for the monitoring of acid ceramidase activity in intact cells, **characterized in that** it comprises:
a) contacting the intact cells with a compound of formula (I) defined in any of claims 1 to 4,
b) washing the cells and adding fresh medium comprising a fluorogenic reporter to obtain stained cells, and
c) washing the cells and measuring the fluorescence emission of the stained cells obtained in step b),
wherein the fluorescence measured is indicative of the acid ceramidase activity

6. A method according to claim 5 wherein the cells are contacted with the compound of formula (I) in step a) between 10 and 240 min.

7. A method according to claim 5 or 6 wherein the concentration of compound of formula (I) in the medium in step a) is between 1 and 50 µM for a number of cells between 20,000 and 1,000,000.

8. A method according to any of claims 5 to 7 wherein the cells are contacted between 1 and 90 min with the fluorogenic reporter.

9. A method according to any of claims 5 to 8 wherein the step c) is carried out by flow cytometry.

10. A method according to any of claims 5 to 9 wherein R₁ is CO-(C3-C20) alkyl.

11. A method according to any of claims 5 to 10 wherein R₂ is a C4-C18 linear alkylene.

12. A method according to any of claims 5 to 11 wherein the fluorogenic reporter is a BODIPY alkynyl derivative, preferably the compound of formula:

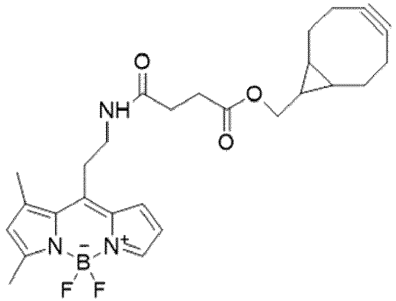

13. Kit for monitoring acid ceramidase activity in intact cells **characterized in that** it comprises a compound of formula (I) as defined in any of claims 1 to 4 and a fluorogenic reporter.

14. Use of the kit defined in claim 13 for *in vitro* monitoring the acid ceramidase activity in intact cells.

15. Use of the kit defined in claim 13 for the diagnosis of a disease caused by the deregulation of ceramidase activity in an isolated sample of a subject, said disease selected from Farber disease and cancer.
